# EUROPEAN PATENT SPECIFICATION

(11) **EP 0 768 072 B1**
(45) Date of publication and mention of the grant of the patent: **04.04.2001**
(21) Application number: 95116271.8
(22) Date of filing: 16.10.1995
(51) Int. Cl.: A61F 13/15

(54) **Compound disposable absorbent article with hump forming element**
Zusammengesetzter saugfähiger Einwegartikel mit erhöhungsformenden Elementen
Article absorbant composite jetable avec un élément formant une protubérance

(43) Date of publication of application: 16.04.1997
(73) Proprietor: THE PROCTER & GAMBLE COMPANY, Cincinnati, Ohio 45202 (US)
(72) Inventor: Wierlacher, Stefan Alois, D-60528 Frankfurt (DE); Lavash, Bruce William, D-61350 Bad Homburg (DE)
(74) Representative: Hirsch, Uwe Thomas

(56) References cited:
- EP-A- 0 302 523
- EP-A- 0 607 985
- WO-A-93/21879
- WO-A-94/16658
- WO-A-96/05790
- DE-A- 4 322 550
- FR-A- 2 653 328
- GB-A- 2 135 892
- US-A- 2 331 355
- US-A- 3 528 422
- US-A- 4 433 972

## Description

### FIELD OF THE INVENTION

The present invention relates to disposable absorbent articles. Disposable absorbent articles are considered to be absorbent devices designed to be worn externally of the body by a user and to receive fluids discharged from the body. In particular the present invention relates to compound disposable absorbent sanitary napkins, catamenials, incontinence inserts and pantiliners comprising a hump forming element to raise the body facing surface of the article towards the perineal area of female users.

### BACKGROUND OF THE INVENTION

In their simplest form, disposable absorbent articles comprise an absorbent element (sometimes referred to as an absorbent core) interposed between a pervious body-contacting element (sometimes referred to as a topsheet or an overwrap) and an impervious protective barrier (sometimes referred to as a backsheet). The absorbent element is, of course, intended to receive and contain the fluids discharged from the body. The body-contacting element is intended to provide more or less comfortable and dry-feeling contact with body surfaces while allowing free passage of fluids therethrough into the absorbent element. The protective barrier is intended to prevent the fluids which are expelled or which escape from the absorbent element from soiling the user's garments.

In addition to the three functional elements mentioned above, disposable absorbent articles are generally provided with means for supporting the device adjacent the user's crotch area, even as the user moves, where it can most effectively perform its intended function. Typically, absorbent articles such as sanitary napkins, catamenials, incontinence inserts and pantiliners are provided with an adhesive attachment means for securing the device to the inner crotch area of the user's undergarments.

While previously known absorbent articles do perform their intended function, each conventional design suffers from certain deficiencies in one or more of absorbency of body fluids, protection of the user's garments from soiling, and/or physical comfort to the user.

With respect to disposable sanitary napkins, at least two general classes presently exist. One such class is identified as being intended for the absorption of medium to high menstrual flows. These sanitary napkins offer a relatively high absorptive capacity. Absorptive capacity is commonly achieved by providing the sanitary napkin with a relatively thick and bulky absorbent member. While having a relatively high absorptive capacity, the bulkiness of the absorbent member may cause a certain degree of wearing discomfort.

A second class of sanitary napkins are intended for light or low menstrual flows and are commonly referred to as pantiliners or pantishields. Sanitary napkins of this class, as a group, are thinner, somewhat more flexible and generally more comfortable than those of the first class. However, sanitary napkins of the second class typically lack the absorptive capacity of sanitary napkins of the first class.

One attempt to provide the benefits of the previously described two classes of sanitary napkins into a single compound sanitary napkin is disclosed in U.S. Patent No. 4,425,130. This compound sanitary napkin comprises a primary menstrual pad and a panty protector joined to one another at their corresponding ends in such a manner that the two constituents are free to move relative to one another along essentially their entire common length. The primary menstrual pad is intended to absorb the bulk of the bodily fluids discharged by the user, while the panty protector is intended to protect the user's garments from soiling. In use, the relative freedom of movement between the primary menstrual pad and the panty protector serves to maintain the primary menstrual pad adjacent the user's crotch region while the panty protector remains associated with the user's undergarment. While the relative freedom of movement between the primary menstrual pad and the panty protector serves to maintain the primary menstrual pad near the user's crotch region, this freedom of movement may lead to a lack of stability if the primary menstrual pad moves laterally beyond the side edges of the panty protector, providing an opportunity for soiling the user's undergarment.

Moreover, the bulky primary menstrual pad, though capable of providing a close body contact, is not sufficiently flexible to mold and conform to the anatomy of the user, and therefore may cause discomfort.

Another attempt to combine the flexibility and the conformability of a thin absorbent article with the close contact with the body provided by a bulky article is disclosed in CA 2154927. The self adapting compound sanitary napkin described in this application comprises a primary absorbent member having a length and a width and a secondary absorbent member having a length and a width. The primary absorbent member and the secondary absorbent member have a common length. The primary absorbent member includes an absorbent core and a fluid pervious topsheet superimposed on said absorbent core. The secondary absorbent member includes a fluid pervious topsheet, a fluid impervious backsheet joined to said topsheet and an absorbent element positioned between the topsheet and the backsheet. The primary absorbent member is affixed to the secondary absorbent member by union means. The width of the secondary absorbent member is preferably at least 1.5 times the width of the primary absorbent member.

While this type of design is effective in that it arranges most of the absorbent material along the centre-line of the pad, where it can absorb promptly the bulk of bodily fluids discharged by the user, it still can have the disadvantage of a poor fit to the anatomy. In an attempt to solve this problem the primary absorbent member may be made bulky enough to stay in intimate contact with the body of the user, or, alternatively, a resilient member may be comprised within the primary absorbent member itself; the resilient member may be constituted by e.g. a fibrous material, or by a hollow and resilient structure.

In any case adding bulkiness to the primary absorbent member does not completely solve the problem of a poor fit to the anatomy of the user since these bulky structures, though capable of pushing the body facing side of the sanitary napkin in close contact with the body of the user, are not flexible enough to mold and to conform effectively and tend to collapse when wet.

Similar designs suffering of the same drawbacks are also described in the following prior art documents.

US 2331355 discloses a sanitary napkin, possibly of layered construction, having a longitudinal central ridge achieved by upwardly gathering a portion of the napkin by means of stitching of adhesive.

EP 302523 discloses a three dimensional unitary sanitary napkin having an inverted-V shaped rear portion. A corresponding thickening in the absorbent core can help create this ridge.

In FR 2653328 a compound sanitary napkin is described which is somewhat similar to that of US 4425130 mentioned above, and having a bulky primary absorbent member superimposed to a thinner secondary absorbent member.

DE 4322550 describes a compound pantiliner having a circular or elliptical shape very short in longitudinal direction, and a pair of side flaps for fixation underneath the crotch of a panty. The pantiliner can comprise two superimposed absorbent elements, and the wearer can discard the uppermost after a certain wearing period while keeping the lowermost for extended usage time.

### SUMMARY OF THE INVENTION

The present invention relates to a compound disposable absorbent article with a hump forming element. The compound disposable absorbent article is intended for wearing adjacent a body discharge area and comprises a body facing surface, a garment facing surface, a longitudinal axis and a lateral axis, a primary absorbent member having a length and a width and a secondary absorbent member having a length and a width. The primary absorbent member and the secondary absorbent member have their length each parallel to the longitudinal axis. The secondary absorbent member is closer to said garment facing surface than the primary absorbent member, and the primary absorbent member is affixed to the secondary absorbent member by joining means. The width of the secondary absorbent member is equal or greater than the width of the primary absorbent member. The article further comprises a hump forming element below the primary absorbent element to raise the body facing surface towards said discharge area. The hump forming element is longer along the longitudinal axis than along the lateral axis, but preferably does not extend beyond the periphery of the primary absorbent member. The maximum height of the hump forming element is preferably greater than the maximum height of the primary absorbent member.

### BRIEF DESCRIPTION OF THE DRAWINGS

While the specification concludes with claims particularly pointing out and distinctly claiming the present invention, it is believed that the present invention will be better understood from the following description in conjunction with the following drawings:
FIG. 1 is a top plan view of one embodiment of a compound sanitary napkin according to the present invention;
FIG. 2 is a cross-sectional view as taken along section line 2-2 of the compound sanitary napkin shown in FIG. 1;
FIG. 3 is a cross-sectional view of the compound sanitary napkin shown in FIGS. 1 and 2 as taken along section line 3-3 of FIG. 2;
FIG. 4 is a top plan view of another embodiment of a compound sanitary napkin according to the present invention;
FIG. 5 is a top plan view of another embodiment of a compound sanitary napkin according to the present invention;
FIG. 6 is a cross-sectional view of another embodiment of a compound sanitary napkin according to the present invention;
FIG. 7 is a cross-sectional view of another embodiment of a compound sanitary napkin according to the present invention;
FIG. 8 is a cross-sectional view of still another embodiment of a compound sanitary napkin according to the present invention.

### DETAILED DESCRIPTION OF THE INVENTION

This invention is of a compound disposable absorbent article which exhibits absorbency for bodily fluids, the protection of the user's garments from soiling, and physical comfort to the user. The compound disposable absorbent article is described below by reference to a sanitary napkin or catamenial. The term "sanitary napkin", as used herein, refers to an article which is worn by females adjacent to the pudendal region and which is intended to absorb and contain the various exudates which are discharged from the body (e.g., blood, menses, and urine) and which is intended to be discarded after a single use.

The term "compound sanitary napkin", as used herein, refers to a sanitary napkin comprised of separate constituents joined to one another to form a unitary structure.

Interlabial devices which reside partially within and partially external of the wearer's vestibule are also within the scope of this invention. As used herein, the term "pudendal" refers to the externally visible female genitalia and is limited to the labia majora, the labia minora, the clitoris, and the vestibule.

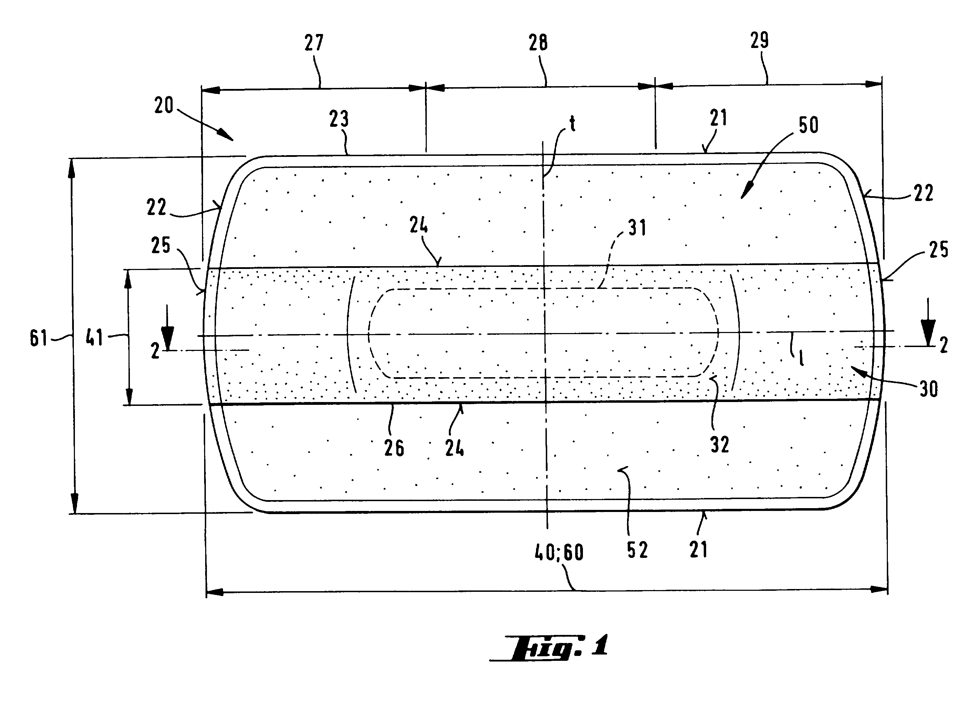

In FIGS. 1-3 one preferred embodiment of a compound sanitary napkin 20 of the present invention is shown. As can be seen in FIGS. 1-3, the compound sanitary napkin 20 comprises a primary absorbent member 30 and a secondary absorbent member 50 affixed together by joining means 70, and a hump forming element 31 comprised between the primary absorbent member 30 and the secondary absorbent member 50.

The compound sanitary napkin has two surfaces, a body contacting or facing surface, and a garment facing or contacting surface. The primary and secondary absorbent members each have corresponding body facing and garment facing surfaces. In use, the secondary absorbent member 50 is intended to stay closer to the garment facing surface of the sanitary napkin 20 than the primary absorbent member 30 which, in turn, is in direct contact with the anatomy of the user. The compound sanitary napkin 20 has two axis, a longitudinal axis and a transverse axis. The term "longitudinal", as use herein, refers to a line, axis or direction in the plane of the compound sanitary napkin that is generally aligned with (e.g., approximately parallel to) a vertical plane which bisects a standing wearer into left and right body halves when the compound sanitary napkin is worn. The terms "transverse" or "lateral", as used herein, are interchangeable, and refer, to a line, axis, or direction which lies within the plane of the compound sanitary napkin that is generally perpendicular to the longitudinal direction.

The primary absorbent member 30 has side edges 24 and end edges 25 which together form the periphery 26 of the primary absorbent member. The secondary absorbent member 50 has side edges 21 and end edges 22 which together form the periphery 23 of the secondary absorbent member and the compound sanitary napkin 20. The compound sanitary napkin 30 has a first end region 27, a central region 28, and a second end region 29.

The primary absorbent member 30 is that constituent of the compound sanitary napkin 20 intended to first receive the bodily fluids discharged by the user. It comprises a fluid receiving layer 33 and a liquid permeable topsheet or coverstock 32 superimposed on the fluid receiving layer 33.

The fluid receiving layer 33 may be constituted by any material that is flexible, conformable and capable of keeping its integrity when wet in order to enhance body fit and comfort of the primary absorbent member; it may be comprised of several different materials including nonwoven or woven webs of synthetic fibres including polyester, polypropylene, or polyethylene; natural fibres including cotton or cellulose; blends of such fibres; or any equivalent materials or combination of materials. In order to enhance the wet integrity of the structure the primary absorbent member 30 should preferably comprise at least 10% by weight, preferably at least 15% by weight of fibres providing wet stability.

Preferably, the primary absorbent member 30 is flexible such that it will deform under relatively small forces that are experienced during normal use. In addition to being flexible, the materials comprising the primary absorbent member 30 are preferably conformable such that the primary absorbent member is able to provide improved fit into and around the labia and perineum. While being generally flexible and conformable under relatively small forces, those forces exerted by the external female genitalia during use, it is also important that the primary absorbent member 30 has sufficient integrity that when subjected to normal wearing forces it does not crumple when wet. Preferably, the primary absorbent member 30 will have sufficient integrity that it will conform to the contours of the body to provide intimate contact with the exposed genitalia of the female user without crumpling or disintegrating when wet. Intimate contact with the exposed female genitalia helps provide better fluid transfer from the user into the primary absorbent member without allowing fluid to bypass and/or run-off the primary absorbent member. While the flexibility and wet integrity characteristics of the primary absorbent member 30 allow for improved fit, they must be balanced against the need for the product to be both soft and comfortable for the wearer. This balancing can be provided by those skilled in the art for example with the aid of trial and error testing with a small group of users.

The topsheet 32 is compliant, soft feeling, and non-irritating to the wearer's skin. Further, the topsheet 32 is liquid pervious, permitting liquid to readily penetrate through its thickness. A suitable topsheet 32 may be manufactured from a wide range of materials such as woven and nonwoven materials; polymeric materials such as apertured formed thermoplastic films, apertured plastic films, and hydroformed thermoplastic films; porous foams; reticulated foams; reticulated thermoplastic films; and thermoplastic scrims. Suitable woven and nonwoven materials can be comprised of natural fibers (e.g., wood or cotton fibers), synthetic fibers (e.g., polymeric fibers such as polyester, polypropylene, or polyethylene fibers); or from a combination of natural and synthetic fibers.

A preferred topsheet comprises an apertured formed film. Apertured formed films are preferred for the topsheet because they are pervious to body exudates and yet non-absorbent and have a reduced tendency to allow liquids to pass back through and rewet the wearer's skin. Thus, the surface of the formed film which is in contact with the body remains dry, thereby reducing body soiling and creating a more comfortable feel for the wearer. Suitable formed films are described in U.S. Pat. No. 3,929,135, issued to Thompson on December 30, 1975; U.S. Pat. No. 4,324,246, issued to Mullane, et al. on April 13, 1982; U.S. Pat. No. 4,342,314, issued to Radel, et al. on August 3, 1982; U.S. Pat. No. 4,463,045, issued to Ahr, et al. on July 31, 1984; and U.S. Pat. No. 5,006,394, issued to Baird on April 9, 1991. The preferred topsheet for the primary absorbent member of the present invention is a formed film described in one or more of the above patents and marketed on sanitary napkins by The Procter & Gamble Company of Cincinnati, Ohio as "DRI-WEAVE".

In a preferred embodiment of the present invention, the body or exposed surface of the formed film topsheet is hydrophilic so as to help liquid transfer through the topsheet faster than if the body surface was not hydrophilic so as to diminish the likelihood that menstrual fluid will flow off the topsheet rather than flowing into and being absorbed by the absorbent core.

The topsheet 32 may be associated with the fluid receiving layer 33 in any suitable manner. Suitable manners include, but are not limited to associating the topsheet 32 with the fluid receiving layer 33 with adhesives such as by spray-gluing or applying lines or spots of adhesives between the topsheet 32 and the fluid receiving layer 33.

Alternatively, or additionally, the topsheet 32 may be associated with the fluid receiving layer 33 by entangling the fibers of the fluid receiving layer 33 with the topsheet 32, by fusing the topsheet 32 to the fluid receiving layer 33 with a plurality of discrete individual fusion bonds.

To insure proper fluid transfer between the topsheet 32 and the fluid receiving layer 33 it is preferred that the topsheet be substantially continuously secured to the underlying fluid receiving layer 33 throughout their common interface. By substantially continuously securing the topsheet 32 to the underlying fluid receiving layer 33 the topsheet 32 will have a reduced tendency to separate from the fluid receiving layer 33 during use. Separation of the fluid receiving layer 33 from the topsheet 32 may inhibit fluid transfer from the topsheet 32 into the underlying fluid receiving layer 33. In the preferred embodiment illustrated in FIG. 3 the fluid receiving layer 33 of the primary absorbent member is completely enwrapped by the topsheet 32.

The hump forming element 31 is positioned below the primary absorbent member 30 and provides the bulkiness necessary to raise the body facing surface of the compound sanitary napkin 20 towards the anatomy of the user; further the hump forming element 31 may preferably be absorbent. The hump forming element 31 may be any means which is generally compressible, resilient, non irritating to the wearer's skin and preferably capable of absorbing and containing body exudates.

The hump forming element 31 may be manufactured from a wide variety of materials commonly used in disposable sanitary napkins, and other disposable absorbent articles, provided they are compressible, resilient and do not collapse when wet. Particularly preferred are those materials, like absorbent foams or absorbent sponges, that are both absorbent and resilient, compressible and do not tend to wet collapse. Suitable absorbent materials comprising foams are described in European Applications EP-A-0 598 833, EP-A-0 598 823 and EP-A-0 598 834.

In the preferred embodiment the total absorbent capacity of the hump forming element 31, together with the other absorbent materials in the primary and secondary should be compatible with the intended exudate loading for the compound sanitary napkin 20. Further, the absorbent capacity of the hump forming element 31 may be varied to accommodate wearers ranging in the expected amount of exudate fluid volume. For instance, a different absorbent capacity may be utilized for compound sanitary napkins intended for day time use as compared with those intended for night time use, or for compound sanitary napkins intended for use by teenage females as compared with those intended by more mature women.

Materials selected for use as the hump forming element 31 are preferably compliant, soft, comfortable, compressible and resilient to enhance body fit and comfort of the compound sanitary napkin while raising the body facing surface of the primary absorbent member 30 toward the discharge area of the user.

While the hump forming element 31 can be generally of any cross-sectional shape in its unstressed condition it is preferably half circular or oval in cross-section. In the embodiment illustrated in FIG. 3, the hump forming element 31 is comprised of an absorbent foam and is manufactured in a generally cylindrical shape with a roughly half oval cross-section.

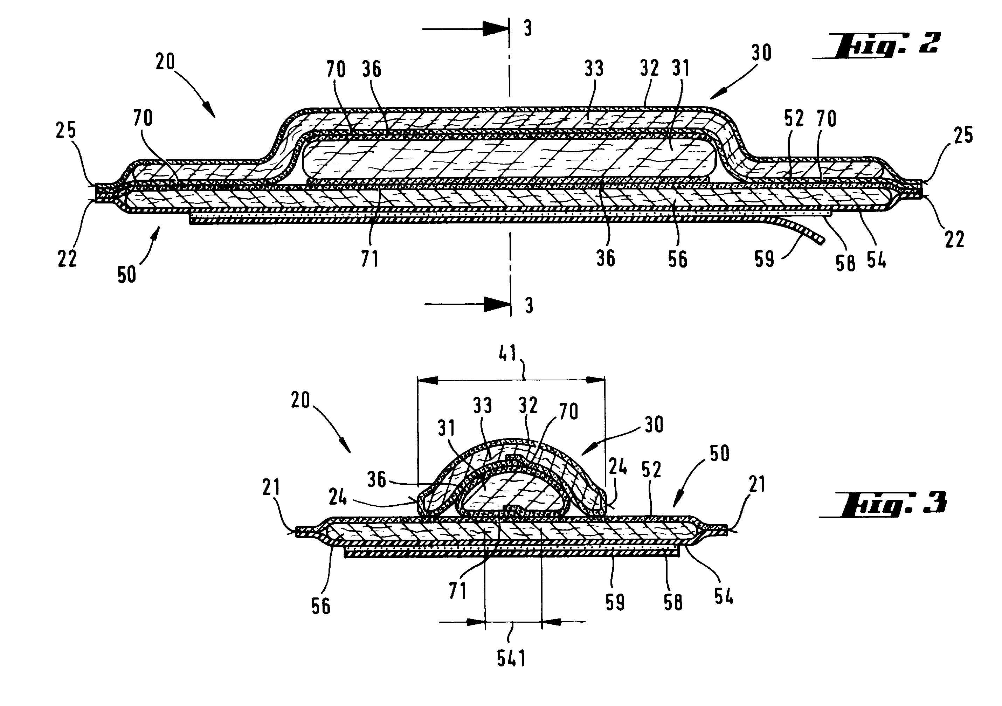

The hump forming element 31 is typically longer along the longitudinal axis of the sanitary napkin than along the lateral axis and does not extend beyond the periphery 26 of the primary absorbent member 30. The hump forming element 31 is preferably positioned so that, in use, it corresponds to the discharge area of the user. As illustrated in FIG. 2 the hump forming element 31 is shorter than the primary absorbent member 30 and extends substantially along the central region 28 of the sanitary napkin.

The hump forming element 31 may have a constant height substantially along its entire length, as illustrated in FIG. 2, or, alternatively, the height of the hump forming element 31 may change along its length. The maximum height of the hump forming element 31 is larger than the maximum height of the primary absorbent member 30 and, preferably, is at least 1.5 times the maximum height of the primary absorbent member 30.

In case the hump forming element 31 is comprised of loose material, e.g. fibrous material, it is preferably enwrapped in a layer 36, which is selected from those described as topsheets above, e.g. a nonwoven layer, as illustrated in FIG. 3.

Referring to FIGS. 1-3, the compound sanitary napkin of the present invention further comprises a secondary absorbent member 50. The secondary absorbent member 50 preferably comprises a liquid permeable topsheet 52, a liquid impervious backsheet 54 joined with the topsheet 52, and an absorbent element 56 positioned between the topsheet 52 and the backsheet 54.

The topsheet 52 can be any fluid pervious material commonly used in sanitary napkins, disposable diapers, and the like. It can be any of the materials described above as being useful in the topsheet 32 of the primary absorbent member 30. The absorbent element 56 can be any absorbent material commonly used in sanitary napkins, disposable diapers, and the like.

As a practical matter, most of the bodily fluids are absorbed by and are contained within the absorbent material of the hump forming element. One major function of the secondary absorbent member 50 is to protect the user's garments from soiling by absorbed fluids which may be expelled from both the primary absorbent member or the hump forming element or which may inadvertently bypass them. Because the absorbent element 56 of the secondary absorbent member 50 performs a different function from that of the absorbent material of the hump forming element 31, the absorbent element 56 can be, and most preferably is, somewhat thinner and less bulky than the hump forming element 31.

Optionally, the secondary absorbent member may be manufactured without an absorbent element. Since in the preferred embodiment most if not all of the bodily fluids are preferably absorbed by and are contained within the absorbent material of the hump forming element 31, the secondary absorbent member 50 need only to protect the user's garments from soiling by relatively small amounts of fluids. Accordingly, an absorbent element may not be necessary to contain the fluids within the secondary absorbent member in order to prevent them from soiling the user's garments.

The backsheet 54 is impervious to liquids (e.g., menses and/or urine) and is preferably manufactured from a thin plastic film, although other flexible liquid impervious materials may also be used. In use, the backsheet 54 is interposed between the absorbent element 56 and the user's undergarments. The function of the backsheet 54 is to prevent exudates which may be expelled from or which inadvertently bypass the hump forming element and exudates absorbed and contained in the absorbent element 56 from contacting and soiling the user's undergarments. The backsheet 54 may thus comprise a woven or nonwoven material, polymeric films such as thermoplastic films of polyethylene or polypropylene, semi-permeable films which provide breathability but prevent liquid transport, or composite materials such as a film-coated nonwoven material. Preferably, the backsheet is a polyethylene film having a thickness of from about 0.012 mm (0.5 mil) to about 0.015 mm (2.0 mil). The backsheet is preferably embossed and/or matte finished to provide a more clothlike appearance. Further, the backsheet may permit vapors to escape from the absorbent element 56 (i.e., breathable) while still preventing exudates from passing through the backsheet.

Preferably, the secondary absorbent member 50 is provided with a support means or attachment means, such as adhesive attachment means 58. The adhesive attachment means 58 provides a means for securing the compound sanitary napkin 20 in the crotch portion of the user's undergarment or panty. The adhesive is typically covered with a removable release liner 59 in order to keep the adhesive from drying out or adhering to a surface other than the crotch portion of the panty prior to use. Any commercially available release liners commonly used for such purposes can be utilized herein. The compound sanitary napkin 20 of the present invention is used by removing the release liner 50 and thereafter placing the sanitary napkin in a panty so that the adhesive 58 contacts the panty. The adhesive 58 maintains the sanitary napkin in its position within the panty during use.

As shown in FIGS. 1-3, the secondary absorbent member can be of generally rectangular shape. Other suitable shapes include but are not limited to oval, hourglass, dog-bone, asymmetric, etc.

Referring now to FIG. 1, the secondary absorbent member 50 preferably has a length 60 and a width 61. The secondary absorbent member is preferably from about 20 to 40 cm long, more preferably from about 25 to 35 cm long, and most preferably is about 30 cm long.

While it can be of generally any cross-section in its unstressed condition, the secondary absorbent member is preferably rectangular in cross-section. The secondary absorbent member is preferably from about 5 to 15 cm in width, more preferably from about 5 to 10 cm in width, and most preferably from about 5 to 8 cm in width. The thickness of the secondary absorbent member 50, as shown in cross-section in FIGS. 2 and 3, is generally less than its width.

Preferably, the secondary absorbent member will be thin and have a caliper of less than about 3.0 millimeters, more preferably less than about 2.6 millimeters, more preferably less than about 2.2 millimeters, and most preferably less than about 2.0 millimeters.

The primary absorbent member of the present invention is thin and flexible due to its lack of bulkiness; it works in combination with the underlying resilient hump forming element that raises the body facing surface of the primary absorbent member towards the discharge area of the user. Due to its thinness and flexibility the primary absorbent member is capable of effectively conform to the anatomy of the user and is provided with the respective shaping force by the underlying hump forming element.

The primary absorbent member and the underlying hump forming element are preferably sized and shaped such that at least a portion of the primary absorbent member will fit within the labia, with the primary absorbent member that comfortably conforms to the anatomy of the user and the hump forming element that provides bulkiness and, preferably, absorbent capacity, while raising the primary absorbent member towards the anatomy of the user.

Accordingly, the width of the primary absorbent member should be sized such that it will reside at least partially within the labia. That is, a portion of the primary absorbent member will preferably fit within the labia during use. Since the exposed female genitalia, including the labia, are generally referred to as soft body tissue, it is important that the materials comprising the primary absorbent member and the hump forming element be comfortable and relative soft such that they are non-irritating and/or uncomfortable for the user. It has been found that a primary absorbent member having a width of about 40 mm and a hump forming element having a width from 15 to 25 mm, preferably of about 15 mm, constitute a preferred combination so that at least part of primary absorbent member will comfortably fit within at least a portion of the labial groove for most women.

While the width of the primary absorbent member is not greater than the width of the secondary absorbent member, it is preferred that the minimum width of the secondary absorbent member is at least 1.25 times the maximum width of the primary absorbent member. More preferably, the minimum width of the secondary absorbent member is at least 1.5 times the maximum width of the primary absorbent member. Most preferably, the minimum width of the secondary absorbent member is about 2 times the maximum width of the primary absorbent member.

Preferably, the secondary absorbent member is about the same length as the primary absorbent member while the compound sanitary napkin is in an unstressed condition. However, it is quite possible for the secondary absorbent member to be longer than the primary absorbent member and still function effectively.

The width of the hump forming element is not greater than the width of the primary absorbent member, and, therefore, it is not greater than the width of the secondary absorbent member as well. It is preferred that the minimum width of the secondary absorbent member is at least 1.5 times the maximum width of the hump forming element. More preferably, the minimum width of the secondary absorbent member is at least 2 times the maximum width of the hump forming element. Most preferably, the minimum width of the secondary absorbent member is in the range from about 3 to about 8 times the maximum width of the hump forming element.

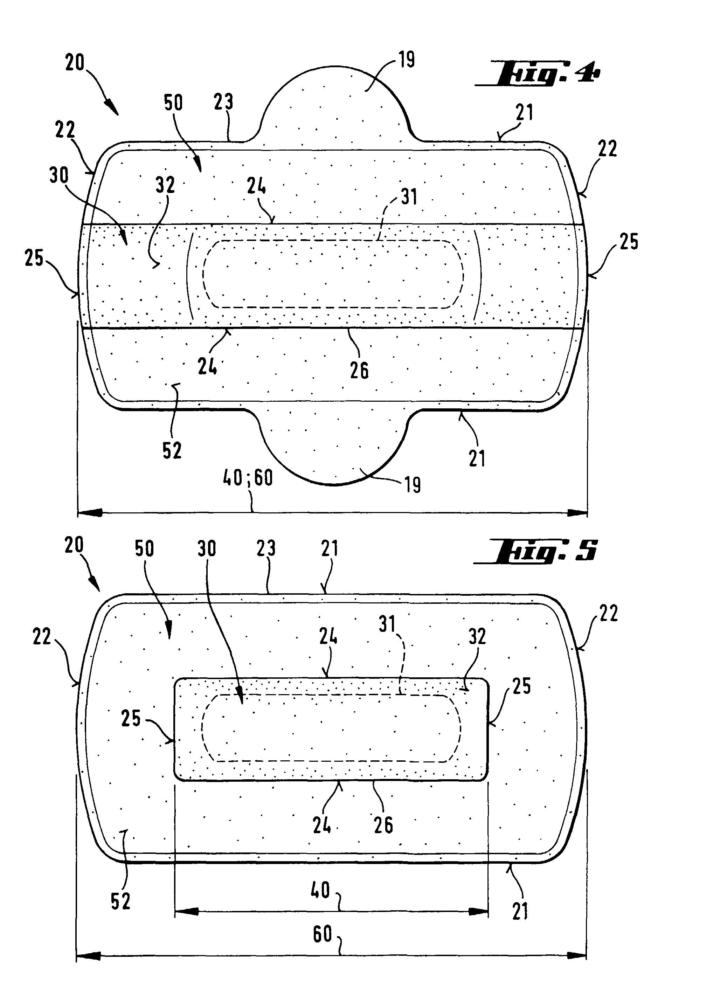

Optionally, the secondary absorbent member 50 may have two flaps 19 each of which are adjacent to and extend laterally from the side edge of the absorbent core, as shown in FIG. 4. The flaps 19 are configured to drape over the edges of the wearer's panties in the crotch region so that the flaps are disposed between the edges of the wearer's panties and the wearer's thighs. The flaps serve at least two purposes. First, the flaps help serve to prevent soiling of the wearer's body and panties by menstrual fluid, preferably by forming a double wall barrier along the edges of the panty. Second, the flaps are preferably provided with attachment means on their garment facing surface so that the flaps can be folded back under the panty and attached to the garment facing side of the panty. In this way, the flaps serve to keep the sanitary napkin properly positioned in the panty.

In a preferred embodiment, the flaps are comprised of the topsheet, absorbent element, and backsheet. Further, the flaps are preferably unitary to the laminae of the secondary absorbent element. In other words, the topsheet, absorbent element, and backsheet simply extend laterally outward to form the flaps. However, the flaps need not be unitary with the secondary absorbent member, but can be separate elements which are affixed to the secondary absorbent member. Further, the flaps can be comprised of a single substrate or other laminae configurations. It is recommended, however, that the flaps have a liquid impervious backsheet to prevent exudates which reach the flaps from soiling the edges of the wearer's panties.

A number of sanitary napkins having flaps suitable or adaptable for use with the secondary absorbent member 50 of the compound sanitary napkin 20 of the present invention are disclosed in U.S. Pat. No. 4,687,478 issued to Van Tilburg on Aug. 18, 1987; U.S. Pat. No. 4,589,876 issued to Van Tilburg on May 20, 1986; and U.S. Pat No. 4,608,047 issued to Mattingly on Aug. 26, 1986.

Optionally, the secondary absorbent member may comprise components that naturally wrap the sides of a wearer's panties. A sanitary napkin having components that naturally wrap the sides of a wearer's panties suitable for use with the secondary absorbent member of the compound sanitary napkin 20 of the present invention are disclosed in U.S. Patent No. 5 584 829, (P&G Case 4961) entitled "Absorbent Article having Panty Covering Components that Naturally Wrap the Sides of Panties", filed July 22, 1993, in the names of Lavash, et al and U.S. Patent No. 5 558 663 (P&G Case 5354) entitled "Absorbent Articles Having Undergarment Covering Components with Zones of Extensibility", filed July 20, 1994, in the names of Weinberger, et al.

The individual components of the primary absorbent member 30 and the secondary absorbent member 50 may be comprised of components that are extensible or elastically stretchable in the longitudinal and/or lateral direction when the compound sanitary napkin is worn.

Referring now to FIGS. 2 and 3, it can be seen that the hump forming element 31 is comprised between the primary absorbent member 30 and the secondary absorbent member 50, symmetrically positioned in transverse direction along the longitudinal axis of the sanitary napkin 20. The hump forming element 31 is affixed to the secondary absorbent member 50 along the longitudinal axis by joining means generally indicated as 71 in FIGS. 2 and 3. It is preferred that the hump forming element 31 is affixed along its whole longitudinal length to the secondary absorbent member 50.

Referring now to FIG. 1, it can be seen that the primary absorbent member 30 and the secondary absorbent member 50 have their length each parallel, and in fact corresponding, to the longitudinal axis of the sanitary napkin 20. To form the compound sanitary napkin of the present invention, the primary absorbent member 30 is affixed to the secondary absorbent member 50 by joining means generally indicated as 70 in FIGS. 2 and 3; preferably the primary absorbent member 30 is not affixed to the hump forming element 31, though there may be discrete points of attachment between them.

The precise nature of the joining means is immaterial so long as the joining means selected serves to join the primary absorbent member to the secondary absorbent member and, optionally but not preferably, to the hump forming member into the compound sanitary napkin of the present invention with sufficient tenacity that the primary absorbent member and the secondary absorbent member are not disconnected during use. Joining means such as adhesive attachment with well known hot melt and pressure sensitive adhesives are quite satisfactory. If the nature of the components selected to construct the constituents of the compound sanitary napkin so permit, heat welding, ultrasonic welding, or a combination of both heat and ultrasonic welding can be used.

Preferably the primary absorbent member is affixed to the secondary absorbent member substantially continuously along the length where they are in direct contact; as illustrated in FIG. 1 and 2 this length corresponds to both end edges 25 of the sanitary napkin and to at least part of the first and second end regions 27 and 29.

The primary absorbent member 30 has a width 41. The compound sanitary napkin has a joining means width 541. The joining means width 541 is less than the width 41 of the primary absorbent member 30. Preferably, the joining means width 541 is less than 75% of the width of the primary absorbent member 30. More preferably, the joining means width 541 is less than 50% of the width of the primary absorbent member 30. Most preferably, the joining means width 541 is less than 25% of the width of the primary absorbent member 30.

Referring now to FIG. 5, there is shown another preferred embodiment of a compound sanitary napkin 20 of the present invention. The primary absorbent member 30 has a length 40 that is less than the length 60 of the secondary absorbent member 50. Any other element of this embodiment is similar to what has been already described referring to FIGS. 1 to 3.

In FIG. 3 it can be seen that topsheet 32 completely encases the fluid receiving layer 33 of the primary absorbent member 30. Also topsheet 36 completely encases the hump forming element 31. In this embodiment, the topsheet 32 for the primary absorbent member 30 is separate and distinct from the topsheet 52 for the secondary absorbent member 50 and topsheet 36 of the hump forming element 31.

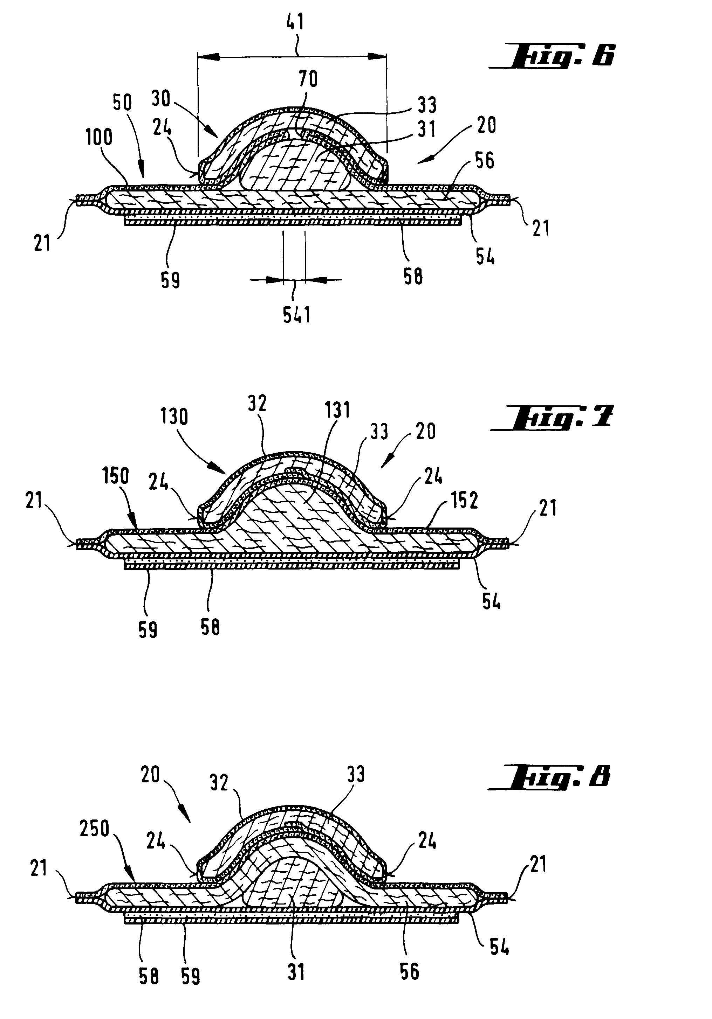

Optionally, the topsheet for the primary absorbent member 30 and the secondary absorbent member 50 may be made of a single web of material, such as topsheet 100 as seen in FIG. 6. In this embodiment topsheet 100 is used for the topsheet on both the primary absorbent member 30 and the secondary absorbent member 50. In the embodiment of FIG. 6 the topsheet 100 can serve as a joining means 70 connecting the primary absorbent member and the secondary absorbent member together. The compound sanitary napkin may also include additional joining means to connect the primary absorbent member to the secondary absorbent member and the hump forming element to the secondary absorbent member. The joining means width 541 is less than the width 41 of the primary absorbent member 30. In the embodiment of FIG. 6 the hump forming element 31 can be provided without topsheet unless topsheet is required to maintain hump forming element 31 with the necessary integrity to provide the desired upward lifting to the primary absorbent member 30.

An alternate embodiment of the present invention is illustrated in FIG. 7, which is similar to that disclosed in FIG. 3, but with the hump forming element 131 integrally formed with the secondary absorbent member 150; a topsheet 152 is superimposed to the common structure constituted by the hump forming element 131 and the secondary absorbent member 150; the primary absorbent member 130 is similar to the primary absorbent member 30 already described with reference to FIG. 3.

A further alternate embodiment of the present invention is illustrated in FIG. 8. The hump forming element 31 is positioned on the garment facing side of the secondary absorbent member 250; more particularly it is comprised between the absorbent element 56 of the secondary absorbent member 250 and the backsheet 54. In this embodiment the hump forming element 31 is provided without topsheet.

It may be desirable to provide a compound sanitary napkin having a primary absorbent member with varying degrees of width or caliper throughout its length. For example, the primary absorbent member may be relatively thicker in the central region as opposed to the end regions. Alternatively, the primary absorbent member may be relatively thinner in the central region as opposed to the end regions.

## Claims

1. A compound disposable absorbent article (20) for wearing adjacent a body discharge area, said article (20) having a body facing surface, a garment facing surface, a longitudinal axis and a lateral axis, a primary absorbent member (30) havinga length (40) and a width (41) and a secondary absorbent member (50) having a length (60) and a width (61), said primary absorbent member (30) and said secondary absorbent member (50) having their length (40, 60) each parallel to said longitudinal axis, said secondary absorbent member (50) being closer to said garment facing surface than said primary absorbent member (30), said primary absorbent member (30) being affixed to said secondary absorbent member (50) by joining means (70), said joining means (70) having a joining means width (541), said width (61) of said secondary absorbent member (50) being greater than said width (41) of said primary absorbent member (30), said article (20) further comprising a hump forming element (31) below said primary absorbent member (30) to raise said body facing surface towards said discharge area,
wherein:
the minimum width of said primary absorbent member (30) is larger than or equal to the maximum width of said hump forming element (31) and the length of said hump forming element (31) is shorter than the length of said primary absorbent member (30).
said joining means width (541) is less than the width (41) of said primary absorbent member (30).

2. A compound disposable absorbent article (20) according to claim 1. characterized in that said joining means (70) are such that said primary absorbent member (30) is not affixed to said hump forming element (31).

3. A compound disposable absorbent article (20) according to any preceding claim, characterized in that the maximum height of said hump forming element (31) is greater than the maximum height of said primary absorbent member (30).

4. A compound disposable absorbent article (20) according to any preceding claim, characterized in that said hump forming element (31) has a maximum height that is at least 1.5 times the maximum height of said primary absorbent member (30).

5. A compound disposable absorbent article (20) according to claim 1, characterized in that said hump forming element (30) is longer along said longitudinal axis than along said lateral axis.

6. A compound disposable absorbent article (20) according to any preceding claim, characterized in that said hump forming element (31) comprises an absorbent foam material.

7. A compound disposable absorbent article (20) according to any preceding claim, characterized in that said primary absorbent member (30) follows the topography of said underlying hump forming element (31).

8. A compound disposable absorbent article (20) according to any preceding claim, characterized in that said primary absorbent member (30) comprises at least 10% by weight, preferably at least 15% by weight of fibres providing wet stability.

9. A compound disposable absorbent article (20) according to any preceding claim, characterized in that said hump forming element (31) is positioned between said primary absorbent member (30) and said secondary absorbent member (50).

10. A compound disposable absorbent article (20) according to any preceding claim, characterized in that the minimum width of said secondary absorbent member (50) is at least 1.25 times, preferably from 1.5 to 2 times, the maximum width of said primary absorbent member (30).

11. A compound disposable absorbent article (20) according to any preceding claim, characterized in that said length (60) of said secondary absorbent member (50) is at least equal to said length (40) of said primary absorbent member (30).

12. A compound disposable absorbent article (20) according to any preceding claim, characterized in that said hump forming element (31) is affixed to said secondary absorbent member (50) along said longitudinal axis, preferably continuously along the whole longitudinal extent of said hump forming element (31).

13. A compound disposable absorbent article (20) according to any preceding claim, characterized in that said hump forming element (31) is integral with said secondary absorbent member (50).

## Patentansprüche

1. Zusammen gesetzter absorbierender Einwegartikel (20) zum Tragen in der Nähe eines Körper-Ausscheidungsbereichs, wobei der Artikel (20) eine körperseitige Oberfläche, eine wäscheseitige Oberfläche, eine längs verlaufende Achse und eine quer verlaufende Achse, ein erstes absorbierendes Teil (30) mit einer Länge (40) und einer Breite (41) und ein zweites absorbierendes Teil (50) mit einer Länge (60) und einer Breite (61) aufweist, wobei das erste absorbierende Teil (30) und das zweite absorbierende Teil (50) mit ihrer Länge (40, 60) jeweils parallel zur längs verlaufenden Achse liegen, wobei das zweite absorbierende Teil (50) näher an der wäscheseitigen Oberfläche als das erste absorbierende Teil (30) liegt, wobei das erste absorbierende Teil (30) mit dem zweiten absorbierenden Teil (50) durch Verbindungsmittel (70) befestigt ist, wobei die Verbindungsmittel (70) eine Verbindungsmittelbreite (541) haben, wobei die Breite (61) des zweiten absorbierenden Teils (50) größer als die Breite (41) des ersten absorbierenden Teils (30) ist, wobei der Artikel (20) ferner ein buckelförmiges Element (31) unterhalb des ersten absorbierenden Teil (30) umfaßt, um die körperseitige Oberfläche in Richtung des Ausscheidungsbereichs erhaben zu machen,
in welchem:
die minimale Breite des ersten absorbierenden Teils (30) größer oder gleich der maximalen Breite des buckelförmigen Elements (31) ist und die Länge des buckelförmigen Elements (31) kürzer als die Länge des ersten absorbierenden Teils (30) ist,
wobei die Verbindungsmittelbreite (541) kleiner als die Breite (41) des ersten absorbierenden Teils (30) ist.

2. Zusammen gesetzter absorbierender Einwegartikel (20) nach Anspruch 1, dadurch gekennzeichnet, daß die Verbindungsmittel (70) derart sind, daß das absorbierende Teil (30) nicht an dem buckelförmigen Element (31) befestigt ist.

3. Zusammen gesetzter absorbierender Einwegartikel (20) nach einem vorstehenden Anspruch, dadurch gekennzeichnet, daß die maximale Höhe des buckelförmigen Elements (31) größer als die maximale Höhe des ersten absorbierenden Teils (30) ist.

4. Zusammen gesetzter absorbierender Einwegartikel (20) nach einem vorstehenden Anspruch, dadurch gekennzeichnet, daß das buckelförmige Element (31) eine maximale Höhe hat, die wenigstens das 1,5-fache der maximalen Höhe des ersten absorbierenden Teils (30) beträgt.

5. Zusammen gesetzter absorbierender Einwegartikel (20) nach Anspruch 1, dadurch gekennzeichnet, daß das buckelförmige Element (31) entlang seiner Längsachse länger als entlang seiner Querachse ist.

6. Zusammen gesetzter absorbierender Einwegartikel (20) nach einem vorstehenden Anspruch, dadurch gekennzeichnet, daß das buckelförmige Element (31) ein absorbierendes Schaummaterial umfaßt.

7. Zusammen gesetzter absorbierender Einwegartikel (20) nach einem vorstehenden Anspruch, dadurch gekennzeichnet, daß das erste absorbierende Teil (30) der Topographie des unterliegenden buckelförmigen Elements (31) folgt.

8. Zusammen gesetzter absorbierender Einwegartikel (20) nach einem vorstehenden Anspruch, dadurch gekennzeichnet, daß das erste absorbierende Teil (30) wenigstens 10 Gew.%, vorzugsweise wenigstens 15 Gew.% Fasern umfaßt, die für ein Nässestabilität sorgen.

9. Zusammen gesetzter absorbierende Einwegartikel (20) nach einem vorstehenden Anspruch, dadurch gekennzeichnet, daß das buckel förmige Element 31) zwischen dem ersten absorbierenden Teil (30) und dem zweiten absorbierenden Teil (50) positioniert ist.

10. Zusammen gesetzter absorbierender Einwegartikel (20) nach einem vorstehenden Anspruch, dadurch gekennzeichnet, daß die minimale Breite des zweiten absorbierenden Teils (50) wenigstens das 1,25-fache, vorzugsweise das 1,5 bis 2-fache, der maximalen Breite des ersten absorbierenden Teils (30) beträgt.

11. Zusammen gesetzter absorbierender Einwegartikel (20) nach einem vorstehenden Anspruch, dadurch gekennzeichnet, daß die Länge (60) des zweiten absorbierenden Teils (50) wenigstens gleich der Länge (40) des ersten absorbierenden Teils (30) ist.

12. Zusammen gesetzter absorbierender Einwegartikel (20) nach einem vorstehenden Anspruch, dadurch gekennzeichnet, daß das buckelförmige Element (31) entlang seiner Längsachse vorzugsweise kontinuierlich entlang der gesamten Längsausdehnung des buckelförmigen Elements (31) mit dem zweiten absorbierenden Teil (50) befestigt ist.

13. Zusammen gesetzter absorbierender Einwegartikel (20) nach einem vorstehenden Anspruch, dadurch gekennzeichnet, daß das buckelförmige Element (31) mit dem zweiten absorbierenden Element (50) einstückig ist.

## Revendications

1. Article absorbant composite jetable (20) destiné à être porté à proximité immédiate d'une zone de décharge corporelle, ledit article (20) présentant une surface faisant face au corps, une surface faisant face au vêtement, un axe longitudinal et un axe latéral, un élément absorbant primaire (30) ayant une longueur (40) et une largeur (41), et un élément absorbant secondaire (50) ayant une longueur (60) et une largeur (61), chaque longueur (40, 60) dudit élément absorbant primaire (30) et dudit élément absorbant secondaire (50) étant parallèle audit axe longitudinal, ledit élément absorbant secondaire (50) étant plus près de ladite surface faisant face au vêtement que ledit élément absorbant primaire (30), ledit élément absorbant primaire (30) étant fixé audit élément absorbant secondaire (50) par un moyen de réunion (70), ledit moyen de réunion (70) ayant une largeur de moyen de réunion (541), ladite largeur (61) dudit élément absorbant secondaire (50) étant supérieure à ladite largeur (41) dudit élément absorbant primaire (30), ledit article (20) comprenant, en outre, un élément formant bosse (31) au-dessous dudit élément absorbant primaire (30) afin d'élever ladite surface faisant face au corps en direction de ladite zone de décharge,
dans lequel
la largeur minimale dudit élément absorbant primaire (30) est plus grande ou égale à la largeur maximale dudit élément formant bosse (31), et la longueur dudit élément formant bosse (31) est plus courte que la longueur dudit élément absorbant primaire (30),
ladite largeur de moyen de réunion (541) est inférieure à la largeur (41) dudit élément absorbant primaire (30).

2. Article absorbant composite jetable (20) selon la revendication 1, caractérisé en ce que ledit élément de réunion (70) est tel que ledit élément absorbant primaire (30) n'est pas fixé audit élément formant bosse (31).

3. Article absorbant composite jetable (20) selon l'une quelconque des revendications précédentes, caractérisé en ce que la hauteur maximale dudit élément formant bosse (31) est plus grande que la hauteur maximale dudit élément absorbant primaire (30).

4. Article absorbant composite jetable (20) selon l'une quelconque des revendications précédentes, caractérisé en ce que ledit élément formant bosse (31) a une hauteur maximale qui est égale au moins à 1,5 fois la hauteur maximale dudit élément absorbant primaire (30).

5. Article absorbant composite jetable (20) selon la revendication 1, caractérisé en ce que ledit élément formant bosse (30) est plus long suivant ledit axe longitudinal que suivant ledit axe latéral.

6. Article absorbant composite jetable (20) selon l'une quelconque des revendications précédentes, caractérisé en ce que ledit élément formant bosse (31) comprend un matériau en mousse absorbant.

7. Article absorbant composite jetable (20) selon l'une quelconque des revendications précédentes, caractérisé en ce que ledit élément absorbant primaire (30) suit la topographie dudit élément formant bosse sous-jacent (31).

8. Article absorbant composite jetable (20) selon l'une quelconque des revendications précédentes, caractérisé en ce que ledit élément absorbant primaire (30) comprend au moins 10% en poids, de préférence au moins 15% en poids de fibres fournissant une stabilité à l'état humide.

9. Article absorbant composite jetable (20) selon l'une quelconque des revendications précédentes, caractérisé en ce que ledit élément formant bosse (31) est placé entre ledit élément absorbant primaire (30) et ledit élément absorbant secondaire (50).

10. Article absorbant composite jetable (20) selon l'une quelconque des revendications précédentes, caractérisé en ce que la largeur minimale dudit élément absorbant secondaire (50) est égale au moins à 1,25 fois, de préférence comprise entre 1,5 et 2 fois la largeur maximale dudit élément absorbant primaire (30).

11. Article absorbant composite jetable (20) selon l'une quelconque des revendications précédentes, caractérisé en ce que ladite longueur (60) dudit élément absorbant secondaire (50) est au moins égale à ladite longueur (40) dudit élément absorbant primaire (30).

12. Article absorbant composite jetable (20) selon l'une quelconque des revendications précédentes, caractérisé en ce que ledit élément formant bosse (31) est fixé audit élément absorbant secondaire (50) suivant ledit axe longitudinal, de préférence de manière continue suivant toute l'étendue longitudinale dudit élément formant bosse (31).

13. Article absorbant composite jetable (20) selon l'une quelconque des revendications précédentes, caractérisé en ce que ledit élément formant bosse (31) est solidaire dudit élément absorbant secondaire (50).
